# EUROPEAN PATENT SPECIFICATION

(11) **EP 1 532 125 B1**
(45) Date of publication and mention of the grant of the patent: **22.03.2006**
(21) Application number: 03761993.9
(22) Date of filing: 24.06.2003
(51) Int. Cl.: C07D 301/10

(54) **A METHOD FOR THE START-UP OF AN EPOXIDATION PROCESS, A CATALYST AND A PROCESS FOR THE EPOXIDATION OF AN OLEFIN**
VERFAHREN ZUM INITIIEREN EINES EPOXIDATIONSVERFAHRENS, KATALYSATOR UND VERFAHREN ZUR EPOXIDATION EINES OLEFINS
PROCEDE DE DEMARRAGE D'UN PROCESSUS D'EPOXYDATION, CATALYSEUR ET PROCEDE D'EPOXYDATION D'UNE OLEFINE

(30) Priority: 28.06.2002 US 392499 P
(43) Date of publication of application: 25.05.2005
(73) Proprietor: SHELL INTERNATIONALE RESEARCH MAATSCHAPPIJ B.V., 2596 HR Den Haag (NL)
(72) Inventor: EVANS, Wayne, Errol, Richmond, TX 77479 (US)
(86) International application number: PCT/US2003/019828
(87) International publication number: WO 2004/002971

(56) References cited:
- EP-A- 0 326 392
- EP-A- 0 352 849
- WO-A-95/05896
- US-A- 2 219 575

## Description

### FIELD OF THE INVENTION

The invention relates to a process for the epoxidation of an olefin, which process comprises contacting a silver-based highly selective epoxidation catalyst with a feed comprising an organic halide.

### BACKGROUND OF THE INVENTION

The catalytic oxidation of olefins over a silver-based catalyst yielding the corresponding olefin oxide has been known for a long time. Conventional silver-based catalysts have provided the olefin oxides with notoriously low selectivity. For example, when using conventional catalysts in the epoxidation of ethylene, the selectivity towards ethylene oxide, expressed as a fraction of the ethylene converted, does not reach values above the 6/7 or 85.7 mole-% limit. Therefore, this limit has long been considered to be the theoretically maximal selectivity of this reaction, based on the stoichiometry of the reaction equation

7 C₂H₄ + 6 O₂ => 6 C₂H₄O + 2 CO₂ + 2 H₂O,

cf. Kirk-Othmer's *Encyclopedia* of *Chemical Technology,* 3^{rd} ed., Vol. 9, 1980, p. 445.

The selectivity determines to a large extent the economical attractiveness of an epoxidation process. For example, one percent improvement in the selectivity of the epoxidation process can reduce the yearly operating costs of a large scale ethylene oxide plant substantially.

The olefin oxide produced by the epoxidation process may be reacted with water, an alcohol or an amine to form a 1,2-diol, a 1,2-diol ether or an alkanolamine. Thus, 1,2-diols, 1,2-diol ethers and alkanolamines may be produced in a multi-step process comprising olefin epoxidation and converting the formed olefin oxide with water, an alcohol or an amine. Any improvement in the selectivity of the epoxidation process can also reduce the yearly operating costs in the overall process for the production of a 1,2-diol, a 1,2-diol ether or an alkanolamine.

Modern silver-based epoxidation catalysts are highly selective towards olefin oxide production. When using the modern catalysts in the epoxidation of ethylene the selectivity towards ethylene oxide can reach values above the 6/7 or 85.7 mole-% limit referred to. Such highly selective catalysts comprise, in addition to silver, a selectivity enhancing dopant which may be selected from rhenium, molybdenum, tungsten and nitrate- or nitrite-forming compounds, cf. for example US-A-4761394 and US-A-4766105.

A reaction modifier, for example an organic halide, may be added to the feed to an epoxidation process for increasing the selectivity (cf. for example EP-A-352850, US-A-4761394 and US-A-4766105). The reaction modifier suppresses the undesirable oxidation of olefin or olefin oxide to carbon dioxide and water, relative to the desired formation of olefin oxide, by a so-far unexplained mechanism. EP-A-352850 teaches that there is an optimum in the selectivity as a function of the concentration of organic halide in the feed, at a constant oxygen conversion level and given set of reaction conditions.

During the initial phase of an epoxidation process, the catalyst experiences the so-called "break-through phase" during which the oxygen conversion is very high, the selectivity is very low, even in the presence of a reaction modifier, and the epoxidation process is difficult to control. It might take a long time in a commercial start-up for the conversion to drop so that the reaction can more easily be controlled at an attractive level of the selectivity. It goes without saying that there is an economical incentive to improve the start-up period and make the catalyst operate at a high selectivity.

US-A-5155242 relates to the start-up of an epoxidation process wherein a conventional catalyst is employed. In this patent document there is disclosed an improved start-up procedure wherein the conventional catalyst is subjected to a pre-soak period in the presence of the organic halide at a temperature less than the operating temperature of the reactor.

US-A-4874879 relates to the start-up of an epoxidation process wherein a highly selective catalyst is employed. In this patent document there is disclosed an improved start-up procedure wherein the highly selective catalyst is subjected to a pre-soak period in the presence of the organic halide at a temperature less than the operating temperature of the reactor.

WO-95/05896 proposes a silver-based catalyst which comprises, as a further component, a selected quantity of chloride. Such catalysts have improved start-up characteristics over catalysts without such chloride.

### SUMMARY OF THE INVENTION

It has unexpectedly been found that the start-up of an epoxidation process wherein a highly selective catalyst is employed can be improved considerably by contacting a pre-soaked highly selective catalyst with a feed gas which does not comprise the organic halide, or comprises the organic halide at a low concentration. The low concentration is typically lower than the concentration at which the catalyst exhibits an optimum selectivity. It is understood that, by doing so, a large portion of the organic halide is stripped off the catalyst, and it has been found that this brings the catalyst at a higher level of selectivity in a subsequent stage of the process during normal olefin oxide production, relative to the case wherein after the pre-soaking the process proceeds without first stripping off organic halide from the catalyst.

Accordingly, the present invention provides a process for the epoxidation of an olefin, which method comprises
(a) pre-soaking a silver-based highly selective epoxidation catalyst with an organic halide, and
(b) passing over the pre-soaked catalyst a feed which is free of organic halide or which comprises an organic halide at a concentration of at most 2×10⁻⁴ mole-%, calculated on the basis of the halogen content relative to the total feed, for a period of more than 16 hours up to 200 hours, and
(c) subsequently contacting the catalyst with a feed comprising the olefin, oxygen and an organic halide wherein the concentration of the organic halide is at least 0.2×10⁻⁴ mole-% higher than the concentration applied in step (b), calculated on the basis of the halogen content relative to the total feed.

The invention also provides a process for producing a 1,2-diol, 1,2-diol ether, or an alkanolamine, comprising converting an olefin oxide into the 1,2-diol, the 1,2-diol ether, or the alkanolamine, wherein the olefin oxide has been obtained by a process for the epoxidation of an olefin according to this invention.

### DETAILED DESCRIPTION OF THE INVENTION

Although the invention may be practiced in many ways, it is preferred to practice it as a gas phase process, i.e. a process in which the feed is contacted in the gas phase with the catalyst which is present as a solid material, typically in a packed bed positioned in a reactor, which is typically a tubular reactor. Frequently, in commercial scale operation, the process of the invention may involve a quantity of catalyst which is at least 10 kg, for example at least 20 kg, frequently in the range of from 10² to 10⁷ kg, more frequently in the range of from 10³ to 10⁶ kg. Generally the process is carried out as a continuous process. The reactor is typically equipped with heat exchange facilities to heat or cool the catalyst. As used herein, the feed is considered to be the composition which is contacted with the catalyst. As used herein, the catalyst temperature or the temperature of the catalyst bed is deemed to be the weight average temperature of the catalyst particles.

As used herein, a highly selective silver-based catalyst is generally a catalyst which, when operated fresh, can exhibit in the gas phase epoxidation of ethylene a theoretical selectivity at zero oxygen conversion, S₀, of at least 6/7 or 85.7 %. More in particular, this theoretical selectivity can be accomplished at a reaction temperature of 260 °C. The value of S₀ for a given catalyst is found by operating the catalyst, in particular at a temperature of 260 °C, in a range of gas hourly space velocities, resulting in a range of selectivity values and oxygen conversion values corresponding to the range of gas hourly space velocities employed. The selectivity values found are then extrapolated back to the theoretical selectivity at zero oxygen conversion, S₀. As used herein, the selectivity is the fraction of the converted olefin which has yielded the olefin oxide.

Generally, the highly selective silver-based catalyst is a supported catalyst. The support may be selected from a wide range of inert support materials. Such support materials may be natural or artificial inorganic materials and they include silicon carbide, clays, pumice, zeolites, charcoal and alkaline earth metal carbonates, such as calcium carbonate. Preferred are refractory support materials, such as alumina, magnesia, zirconia and silica. The most preferred support material is α-alumina.

The support material is preferably porous and has preferably a surface area, as measured by the B.E.T. method, of at most 20 m²/g and in particular from 0.05 to 20 m²/g. More preferably the B.E.T. surface area of the support is in the range of 0.1 to 10, in particular from 0.1 to 3.0 m²/g. As used herein, the B.E.T. surface area is deemed to have been measured by the method as described in Brunauer, Emmet and Teller in *J. Am. Chem. Soc.* 60 (1938) 309-316.

Generally, the highly selective silver-based catalysts comprise, in addition to silver, a Group IA metal, and one or more selectivity enhancing dopants selected from rhenium, molybdenum, tungsten, and a sulfate-, nitrate- or nitrite-forming compound. Silver is suitable present in a quantity of from 10 to 500 g/kg on the total catalyst. The Group IA°metals, as well as the selectivity enhancing dopants, may each be present in a quantity of from 0.01 to 500 mmole/kg, calculated as the element (rhenium, molybdenum, tungsten, Group IA metal, sulfur or nitrogen) on the total catalyst. The sulfate-forming compound may suitably be a sulfate, for example a sulfate of a Group IA metal. The nitrate- or nitrite-forming compounds and particular selections of nitrate- or nitrite-forming compound are as defined hereinafter. Preferably, the Group IA metal is selected from lithium, potassium, rubidium and cesium. Rhenium, molybdenum, tungsten, or the sulfate-, nitrate- or nitrite-forming compound may suitably be provided as an oxyanion, for example, as a perrhenate, molybdate, tungstate, sulfate, nitrate or nitrate, in salt or acid form.

Of special preference are the silver-based catalysts which comprise rhenium, in addition to silver. Such catalysts are known from US-A-4761394 and US-A-4766105, which are incorporated herein by reference. Broadly, they comprise silver, rhenium or compound thereof, a further metal or compound thereof and optionally a rhenium co-promoter which may be selected from one or more of sulfur, phosphorus, boron, and compounds thereof, on the support material. More specifically the further metal is selected from the group of Group IA metals, Group IIA metals, molybdenum, tungsten, chromium, titanium, hafnium, zirconium, vanadium, thallium, thorium, tantalum, niobium, gallium and germanium and mixtures thereof. Preferably the further metal is selected from the Group IA metals such as lithium, potassium, rubidium and cesium and/or from the Group IIA metals such as calcium and barium. Most preferably it is lithium, potassium and/or cesium. Where possible, rhenium, the further metal or the rhenium co-promoter is provided as an oxyanion, in salt or acid form.

Preferred amounts of the components of these catalysts are, when calculated as the element on the total catalyst:
- silver from 10 to 500 g/kg,
- rhenium from 0.01 to 50 mmole/kg,
- the further metal or metals from 0.1 to 500 mmole/kg each, and, if present,
- the rhenium co-promoter or co-promoters from 0.1 to 30 mmole/kg each.

The preparation of the catalysts is known in the art and the known methods are applicable to this invention. Methods of preparing the catalyst include impregnating the support with a silver compound and with other catalyst ingredients, and performing a reduction to form metallic silver particles. Reference may be made, for example, to US-A-4761394, US-A-4766105, US-A-5380697, US-A-5739075, US-B1-6368998, US-2002/0010094 A1, WO-00/15333, WO-00/15334 and WO-00/15335, which are incorporated herein by reference. Suitable catalysts for use in this invention are, for example, the S-879, S-881 and S-882 catalysts, commercially available from CRI Catalyst Company.

This invention may be used with new catalysts, as well as aged catalysts which, due to a plant shut-down, have been subjected to a prolonged shut-in period.

The olefin for use in the epoxidation process may be any olefin, such as an aromatic olefin, for example styrene, or a di-olefin, whether conjugated or not, for example 1,9-decadiene or 1,3-butadiene. Typically, the olefin is a monoolefin, for example 2-butene or isobutene. Preferably, the olefin is a mono-α-olefin, for example 1-butene or propylene. The most preferred olefin is ethylene.

The epoxidation process may be air-based or oxygen-based, see Kirk-Othmer's *Encyclopedia of Chemical Technology,* 3rd ed., Vol. 9, 1980, p. 445-447. In the air-based process air or air enriched with oxygen is employed as the source of the oxidizing agent while in the oxygen-based processes high-purity (>95 mole-%) oxygen is employed as the source of the oxidizing agent. Presently most epoxidation plants are oxygen-based and this is a preferred embodiment of the present invention.

Oxygen is typically applied in the epoxidation process at a concentration which avoids the flammable regime. The concentration of oxygen in the feed may be adjusted as the concentration of the olefin is changed so as to remain outside the flammable regime. The actual safe operating ranges depend, along with the feed composition, also on the epoxidation conditions such as the catalyst temperature and the pressure.

The organic halide, as the reaction modifier, is in particular an organic bromide, and more in particular an organic chloride. Preferred organic halides are chlorohydrocarbons or bromohydrocarbons. More preferably they are selected from the group of methyl chloride, ethyl chloride, ethylene dichloride, ethylene dibromide, vinyl chloride or a mixture thereof. Most preferred reaction modifiers are ethyl chloride and ethylene dichloride.

Although the organic halide may be supplied as a single compound, upon contact with the catalyst a variety of compounds may be formed which function as reaction modifier, and which may be present in the feed if a recycle is applied. For example, when applying ethyl chloride in an ethylene oxide process, the feed may in practice comprise ethyl chloride, vinyl chloride, ethylene dichloride and methyl chloride.

In preferred embodiments, organic halide is employed as the single reaction modifier. In other embodiments, nitrate- or nitrite-forming compounds, e.g. nitrogen oxides and/or organic nitrogen compounds, are used as reaction modifier together with the organic halide, in particular an organic chloride. Suitable nitrogen oxides are of the general formula NOₓ wherein x, which denotes the ratio of the number of oxygen atoms to the number of nitrogen atoms, is in the range of from 1 to 2. These nitrogen oxides include for example NO, N₂O₃ and N₂O₄. Suitable organic nitrogen compounds are nitro compounds, nitroso compounds, amines, nitrates and nitrites, for example nitromethane, 1-nitropropane or 2-nitropropane. Hydrazine, hydroxylamine or ammonia may be employed as well. It is frequently considered that under the operating conditions of olefin epoxidation the nitrogen containing reaction modifiers are precursors of nitrates or nitrites, i.e. they are so-called nitrate- or nitrite-forming compounds (cf. e.g. EP-A-3642, US-A-4822900).

In the epoxidation process the feed may contain one or more optional components, such as carbon dioxide, inert gases and saturated hydrocarbons. Carbon dioxide is a by-product in the epoxidation process. However, carbon dioxide generally has an adverse effect on the catalyst activity, and high concentrations of carbon dioxide are therefore typically avoided. The inert gas may be, for example, nitrogen or argon, or a mixture thereof. Suitable saturated hydrocarbons are propane and cyclopropane, and in particular methane and ethane. Saturated hydrocarbons may be added to the feed in order to increase the oxygen flammability limit.

When new catalysts are utilised in the epoxidation process, it has been found useful in some instances to pretreat these catalysts prior to carrying out the start-up method by subjecting them to a high temperature with a sweeping gas passing over the catalyst. The sweeping gas is typically an inert gas, for example nitrogen or argon, or mixtures comprising nitrogen and/or argon. The high catalyst temperature converts a significant portion of organic nitrogen compounds which may have been used in the manufacture of the catalysts to nitrogen containing gases which are swept up in the gas stream and removed from the catalyst. In addition, any moisture may be removed from the catalyst. Typically, when the catalyst is loaded in the reactor, by utilizing the coolant heater, the temperature of the catalyst is brought up to 200 to 250 °C and the gas flow is passed over the catalyst. The start-up of used catalysts may or may not require the use of a sweeping gas, but it may frequently be used. Further details on these procedures may be found in US-A-4874879, which is incorporated herein by reference.

As indicated hereinbefore, the practice of this invention involves the steps of pre-soaking the catalyst with an organic halide and then contacting the catalyst with a feed comprising an organic halide at a low concentration, if any.

The pre-soaking of the catalyst may be accomplished by contacting the catalyst with a feed comprising the organic halide. For the sake of clarity only, this step of the process will be indicated hereinafter by the term "pre-soak phase". In the pre-soak phase, the quantity of the organic halide contacted with the catalyst may typically be at most 100 mmole/kg catalyst, more typically in the range of from 0.01 to 50 mmole/kg catalyst, in particular from 0.05 to 20 mmole/kg catalyst, more in particular from 0.1 to 10 mmole/kg catalyst, calculated on the basis of the halogen content of the organic halide. The concentration of the organic halide in the feed may be at least 1.5×10⁻⁴ mole-%, more typically at least at least 2×10⁻⁴ mole-%, in particular at least 2.5×10⁻⁴ mole-%, calculated as moles of halogen, relative to the total feed. The concentration of the organic halide in the feed may be, for example, up to 0.1 mole-%, relative to the total feed, for example up to 0.01 mole-%, calculated as moles of halogen, relative to the total feed. The organic halide is preferably added slowly, typically over a period of several hours, for example, over a period of from 1 to 30 hours, more typically 2 to 20 hours. These times and concentrations, however, are not material to the invention and higher or lower values of the concentration and time may be applied. Generally, the concentration of the organic halide in the feed is higher during the pre-soak phase than during the subsequent stripping phase, as described hereinafter. During the pre-soak phase, the catalyst temperature is typically at most 270 °C, preferably in the range of from 180 to 265 °C, more preferably in the range of from 200 to 260 °C, inclusive. Typically, the reactor inlet pressure is at most 4000 kPa absolute, more typically at most 3500 kPa, most typically at most 2500 kPa absolute: Typically, the reactor inlet pressure is at least 500 kPa absolute. "GHSV" or Gas Hourly Space Velocity is the unit volume of gas at normal temperature and pressure (0 ºC, 1 atm, i.e. 101.3 kPa) passing over one unit volume of packed catalyst per hour. Preferably, when the present invention is practiced as a gas phase process involving a packed catalyst bed, the GHSV is in the pre-soak phase in the range of from 500 to 10000 Nl/(l.h).

Subsequent to the pre-soak phase, a feed is passed over the catalyst which is free of the organic halide, or may comprise organic halide at a low concentration. When the invention is practiced using a reactor which is equipped with a recycle loop, and no external source of organic halide is employed in this stage of the process, the feed may still contain organic halide as a result of the existence of a recycle stream. The low concentration of organic halide may be at most 2×10⁻⁴ mole-%, more typically it is at most 1.5×10⁻⁴ mole-%, calculated on the basis of the halogen content relative to the total feed. When practicing this invention, the low concentration of the organic halide is frequently at least 0.01×10⁻⁴ mole-%, on the same basis. This step may be considered the stripping phase of the process, and, for the sake of clarity only, this step of the process will be indicated hereinafter by the term "stripping phase". During the stripping phase, the catalyst temperature is typically at most 270 °C, preferably in the range of from 200 to 265 °C, more preferably in the range of from 220 to 260 °C, inclusive. Typically, the reactor inlet pressure is at most 4000 kPa absolute, more typically at most 2500 kPa absolute, most typically at most 2500 kPa absolute. Typically, the reactor inlet pressure is at least 500 kPa absolute. Preferably, when the present invention is practiced as a gas phase process involving a packed catalyst bed, the GHSV is in the stripping phase in the range of from 500 to 10000 Nl/(l.h). Typically, these conditions may be maintained for a period of from 20 to 60 hours. Typically, the reactor inlet pressure and the GHSV will not be changed when leaving the pre-soak phase.

During the stripping phase the feed comprises, preferably, one or more components selected from the olefin, oxygen, carbon dioxide, the inert gases and additional reaction modifiers, such as a nitrate- or nitrite-forming compound. However, the presence of one or more of these additional components in the feed during the stripping phase is not considered to be essential to the invention.

In a particular embodiment, amongst others, the start-up section of the process may include prior to the stripping phase several steps as follows. Firstly, the catalyst may be contacted with a feed comprising the olefin and optionally a saturated hydrocarbon, in particular ethylene and optionally methane. The organic halide may then be added to the feed, to initiate the pre-soak phase, as described hereinbefore. When the desired quantity of organic halide has been added, oxygen may be added to the feed. As an alternative, oxygen may be added to the feed simultaneously or shortly after the first addition of the organic halide to the feed. Within a few minutes of the addition of oxygen the epoxidation reaction may initiate.

In this stage of the particular embodiment of the previous paragraph, with the olefin, the saturated hydrocarbon (optional), the organic halide and oxygen being added to the feed, the feed comprises typically the olefin in a quantity of from 5 to 70 mole-%, more typically from 10 to 50 mole-%; the saturated hydrocarbon in a quantity of from 0 to 70 mole-%, more typically from 10 to 60 mole-%; and oxygen in a quantity of from 0.5 to 15 mole-%, more typically from 1 to 12 mole-%. An advantage of applying this particular embodiment is that the heat of formation of the olefin oxide assists in controlling the catalyst temperature. It may be advantageous to apply during the start-up a lower oxygen concentration and a lower olefin concentration in the feed, compared with the feed composition in later stages of the process during normal olefin oxide production. Lower oxygen concentration and a lower olefin concentration in the feed will reduce the oxygen conversion level so that, advantageously, hot spots in the catalyst are better avoided and the process will be more easily controllable.

After the stripping phase, the concentration of organic halide in the feed is increased, typically to a value which is practical for the production of the olefin oxide with high selectivity, and in particular to a value at which the selectivity to the olefin oxide is at its optimum. The increase in the organic halide concentration in the feed may be at least 0.2×10⁻⁴ mole-%, typically at least 0.5×10⁻¹⁴ mole-%, in particular at least 1×10⁻⁴ mole-%, more in particular at least 2×10⁻⁴ mole-%, calculated as moles of halogen, relative to the total feed. For the sake of clarity only, the phase of the epoxidation process after the stripping phase, i.e. the phase of the process in which normal olefin oxide production is achieved, will be indicated herein by the term "post-stripping phase". In the post-stripping phase, the invention may be practiced by using methods known in the art of epoxidation processes. Reference may be made, for example to US-A-4761394, US-A-4766105, US-B1-6372925, US-A-4874879 and US-A-5155242, which are incorporated herein by reference.

In the post-stripping phase, the catalyst temperature is typically in the range of from 180 to 260°C, more typically in the range of from 200 to 255 °C. Such temperatures are in particular suitable as long as the catalyst has not yet substantially been subject to an aging-related performance decline. Such aging manifests itself by a reduction in the activity of the catalyst. When a reduction in activity of the catalyst is manifest, the catalyst temperature may be increased in order to compensate for the reduction in activity. The catalyst temperature may ultimately be increased, for example, up to a temperature of 325 °C, typically in the range of from 270 to 300 °C. Generally speaking, the catalyst temperature may be increased until it becomes undesirably high, at which point in time the catalyst is deemed to be at the end of its lifetime and would need to be exchanged.

In the post-stripping phase, the concentration of the components in the feed may be selected within wide ranges, as described hereinafter.

Typically, the olefin concentration in the feed is in the range of from 5 to 70 mole-%, in particular from 10 to 50 mole-%, relative to the total feed. If desired, the olefin concentration may be increased during the lifetime of the catalyst, by which the selectivity may be improved in an operating phase wherein the catalyst has aged (cf. US-B1-6372925, which is incorporated herein by reference).

Typically, the concentration of oxygen is within the range of from 1 to 15 mole-%, more typically from 2 to 12 mole-% of the total feed.

Typically, concentrations of carbon dioxide in the feed in excess of 20 mole-%, preferably in excess of 10 mole-%, more preferably in excess of 5 mole-%, relative to the total feed, are avoided during the epoxidation process and its start-up. A concentration of carbon dioxide as low as 1 mole-% or lower, relative to the total feed, may be employed. Inert gas may be present in the feed in a concentration of from 0.5 to 95 mole-%. In an air based process inert gas may be present in the feed in a concentration of from 30 to 90 mole-%, typically from 40 to 80 mole-%. In an oxygen based process inert gas may be present in the feed in a concentration of from 0.5 to 30 mole-%, typically from 1 to 15 mole-%. If saturated hydrocarbons are present, they may be present in a quantity of up to 70 mole-%, typically in a quantity of from 10 to 60 mole-%, relative to the total feed.

The concentration of the organic halide in the feed may be at least 1×10⁻⁴ mole-%, more typically at least at least 1.5×10⁻⁴ mole-%, in particular at least 2×10⁻⁴ mole-%, more in particular at least 2.5×10⁻⁴ mole-%, calculated as moles of halogen, relative to the total feed. The organic halides are generally effective as reaction modifier when used in low concentration in the feed, for example up to 0.1 mole-%, relative to the total feed, for example up to 0.01 mole-%, calculated as moles of halogen, relative to the total feed. In particular when the olefin is ethylene, it is preferred that the organic halide is present in the feed at a concentration of up to 50×10⁻⁴ mole-%, in particular up to 30×10⁻⁴ mole-%, more in particular up to 10×10⁻⁴ mole-%, calculated as moles of halogen, relative to the total feed. When nitrogen containing reaction modifiers are applied, they may be present in low concentration in the feed, for example up to 0.1 mole-%, calculated as moles of nitrogen, relative to the total feed, for example from 0.01×10⁻⁴ to 0.01 mole-%. In particular when the olefin is ethylene, it is preferred that the nitrogen containing reaction modifier is present in the feed at a concentration of from 0.05×10⁻⁴ to 50×10⁻⁴ mole-%, in particular from 0.2×10⁻⁴ to 30×10⁻⁴ mole-%, more in particular from 0.5×10⁻⁴ to 10×10⁻⁴ mole-%, calculated as moles of nitrogen, relative to the total feed.

At any moment of the post-stripping phase, the concentration of the organic modifier in the feed may be adjusted so as to achieve an optimal selectivity towards the olefin oxide formation.

In the post-stripping phase, further reaction conditions may be selected from wide ranges, as set out hereinafter. The reactor inlet pressure is typically at most 4000 kPa absolute, more typically at most 3500 kPa absolute, most typically at most 2500 kPa absolute. Typically, the reactor inlet pressure is at least 500 kPa absolute. Preferably, when the epoxidation process is carried out as a gas phase process involving a packed catalyst bed, the GHSV is in the range of from 1500 to 10000 Nl/(l.h). Typically, the reactor inlet pressure and the GHSV will not be changed when leaving the stripping phase. Preferably, the work rate is in the range of from 0.5 to 10 kmole olefin oxide produced per m³ of catalyst per hour, in particular 0.7 to 8 kmole olefin oxide produced per m³ of catalyst per hour, for example 5 kmole olefin oxide produced per m³ of catalyst per hour. As used herein, the work rate is the amount of the olefin oxide produced per unit volume of catalyst per hour and the selectivity is the molar quantity of the olefin oxide formed relative to the molar quantity of the olefin converted.

The olefin oxide produced may be recovered from the reactor product by using methods known in the art, for example by absorbing the olefin oxide from a reactor outlet stream in water and optionally recovering the olefin oxide from the aqueous solution by distillation. At least a portion of the aqueous solution containing the olefin oxide may be applied in a subsequent process for converting the olefin oxide into a 1,2-diol or a 1,2-diol ether.

The olefin oxide produced in the present epoxidation process may be converted into a 1,2-diol, a 1,2-diol ether, or an alkanolamine. As this invention leads to a more attractive process for the production of the olefin oxide, it concurrently leads to a more attractive process which comprises producing the olefin oxide in accordance with the invention and the subsequent use of the obtained olefin oxide in the manufacture of the 1,2-diol, 1,2-diol ether and/or alkanolamine.

The conversion into the 1,2-diol or the 1,2-diol ether may comprise, for example, reacting the olefin oxide with water, suitably using an acidic or a basic catalyst. For example, for making predominantly the 1,2-diol and less 1,2-diol ether, the olefin oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-diol ethers in the reaction mixture is increased. The 1,2-diol ethers thus produced may be a di-ether, tri-ether, tetra-ether or a subsequent ether. Alternative 1,2-diol ethers may be prepared by converting the olefin oxide with an alcohol, in particular a primary alcohol, such as methanol or ethanol, by replacing at least a portion of the water by the alcohol.

The conversion into the alkanolamine may comprise, for example, reacting the olefin oxide with ammonia. Anhydrous or aqueous ammonia may be used, although anhydrous ammonia is typically used to favour the production of monoalkanolamine. For methods applicable in the conversion of the olefin oxide into the alkanolamine, reference may be made to, for example US-A-4845296, which is incorporated herein by reference.

The 1,2-diol and the 1,2-diol ether may be used in a large variety of industrial applications, for example in the fields of food, beverages, tobacco, cosmetics, thermoplastic polymers, curable resin systems, detergents, heat transfer systems, etc. The alkanolamine may be used, for example, in the treating ("sweetening") of natural gas.

Unless specified otherwise, the organic compounds mentioned herein, for example the olefins, 1,2-diols, 1,2-diol ethers and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

The following example will illustrate the invention, without limiting the scope of the invention.

### EXAMPLE

A catalyst, as defined in US-A-4766105, comprising silver, rhenium and cesium on an α-alumina support was employed in the following experiment.

A tubular microreactor was charged with a 4.2-g sample of the crushed catalyst. The catalyst in the reactor was heated at 215 °C during 40 hours under a flow of nitrogen gas at GHSV of 820 Nl/l.h. The catalyst temperature was increased to 225 °C, the nitrogen feed to the catalyst was replaced by a feed of an ethylene/carbon dioxide/nitrogen mixture, and subsequently ethyl chloride was added to the feed. Then oxygen was added to the feed. The oxygen/ethylene/carbon dioxide/nitrogen volume ratio in the resulting feed amounted to 4:15:4:77. The ethyl chloride concentration in the feed was 2.6 ppmv (i.e. 2.6×10⁻⁴ mole-%, calculated as moles of chlorine, relative to the total feed). The GHSV was 3300 Nl/l.h. The reactor inlet pressure was 1530 kPa absolute. These conditions were maintained for 2 hours.

The catalyst was then subjected to changing reaction conditions, as set out in Table I.

**Table I**

| Step | Runhour | Catalyst temperature (°C) | Ethyl chloride in feed (ppmv) | Time (h) |
|---|---|---|---|---|
| 1*) | 1 | 225 | 2.6 | 2 |
| 2 | 3 | 235 | 2.6 | 2 |
| 3 | 5 | **) | 2.6 | 8 |
| 4 | 13 | 245 | 2.4 | 1 |
| 5 | 14 | 248 | 2.1 | 1 |
| 6 | 15 | 250 | 1.8 | 1 |
| 7 | 16 | 253 | 1.5 | 1 |
| 8 | 17 | 255 | 1.2 | 1 |
| 9 | 18 | 258 | 1.0 | 1 |
| 10 | 19 | 260 | 0.9 | 48 |
| 11 | 67 | 250 | 1.2 ***) | 2 |
| 12 | 69 | 250 | 1.8 | 2 |
| 13 | 71 | 250 | 2.2 | 2 |
| 14 | 73 | < 250 **) | 2.2 ' | 20 |
| 15 | 93 | < 250 **) | 2.6 | 16 |
| 16 | 109 | < 250 **) | 3.0 | 10 |
| 17 | 119 | < 250 **) | 2.6 | Hold |
| *) The temperature increase to 225 °C defined the beginning of Runhour 1 | | | | |
| **) The catalyst temperature was adjusted so as to obtain and maintain 3.1 %v ethylene oxide in the reactor outlet stream; steps 14-16 involved an optimization of the selectivity at constant ethylene oxide content (3.1 %v) in the reactor outlet stream | | | | |
| ***) The feed composition was changed to oxygen/ethylene/carbon dioxide/nitrogen volume ratio 8/30/2/60 | | | | |

The temperature of the catalyst at the beginning of step 17 was 246 °C, the selectivity was 86.5 %-mole.

## Claims

1. A process for the epoxidation of an olefin, which process comprises the steps of
(a) pre-soaking a silver-based highly selective epoxidation catalyst with an organic halide,
(b) passing over the pre-soaked catalyst a feed which is free of organic halide or which comprises an organic halide at a concentration of at most 2×10⁻⁴ mole-%, calculated on the basis of the halogen content relative to the total feed, for a period of more than 16 hours up to 200 hours, and
(c) subsequently contacting the catalyst with a feed comprising the olefin, oxygen and an organic halide wherein the concentration of the organic halide is at least 0.21×10⁻⁴ mole-% higher than the concentration applied in step (b), calculated on the basis of the halogen content relative to the total feed.

2. A method as claimed in claim 1, wherein the catalyst comprises, in addition to silver, a Group IA metal, and one or more selectivity enhancing dopants selected from rhenium, molybdenum, tungsten, and a sulfate, nitrate- or nitrite-forming compound.

3. A method as claimed in claim 2, wherein the catalyst comprises, in addition to silver, rhenium or compound thereof, a further metal or compound thereof selected from the group of Group IA metals, Group IIA. metals, molybdenum, tungsten, chromium, titanium, hafnium, zirconium, vanadium, thallium, thorium, tantalum, niobium, gallium and germanium and mixtures thereof, and optionally a rhenium co-promoter which may be selected from one or more of sulfur, phosphorus, boron, and compounds thereof, on a support material.

4. A method as claimed in any of claims 1-3, wherein the organic halide comprises one or more chlorohydrocarbons or bromohydrocarbons.

5. A method as claimed in any of claims 1-4, wherein the catalyst is pre-soaked by contacting the catalyst with a quantity of the organic halide in the range of from 0.01 to 50 mmole/kg catalyst, in particular from 0.05 to 20 mmole/kg catalyst, more in particular from 0.1 to 10 mmole/kg catalyst, calculated on the basis of the halogen content of the organic halide, at a temperature in the range of from 180 to 265 °C, more preferably in the range of from 200 to 260 °C, inclusive.

6. A method as claimed in any of claims 1-5, wherein the feed passing over the pre-soaked catalyst comprises the organic halide at a concentration in the range of from 0.01×10⁻⁴ to at most 1.5×10⁻⁴ mole-%, calculated on the basis of the halogen content relative to the total feed.

7. A method as claimed in any of claims 1-6, wherein the period is in the range of from 20 to 60 hours.

8. A method as claimed in any of claims 1-7, wherein the temperature is at most 270 °c, in particular in the range of from 200 to 265 °C, when the feed is passed over the pre-soaked catalyst.

9. A method as claimed in any of claims 1-8, wherein the feed passing over the pre-soaked catalyst comprises an olefin, in particular ethylene, optionally a saturated hydrocarbon, and oxygen, and wherein the quantity of the olefin is in the range of from 5 to 70 mole-%, in particular from 10 to 50 mole-%; the quantity of the saturated hydrocarbon is in the range of from 0 to 70 mole-%, in particular from 10 to 60 mole-%; and the quantity of oxygen is in the range of from 0.5 to 15 mole-%, in particular from 1 to 12 mole-%.

10. A process as claimed in any of claims 1-9, wherein in step (c) the concentration of the organic halide is at least 1×10⁻⁴ mole-% higher than the concentration, applied in step (b), calculated on the basis of the halogen content relative to the total feed.

11. A process as claimed in any of claims 1-10, wherein in step (c) the feed comprises the organic halide at concentration at which the selectivity to the olefin oxide is at its optimum.

12. A process as claimed in any of claims 1-11, wherein the olefin is ethylene.

13. A process for producing a 1,2-diol, 1,2-diol ether, or an alkanolamine, comprising concerting an olefin oxide into the 1,2-diol, the 1,2-diol ether, or the alkanolamine, wherein the olefin oxide has been obtained by a process for the epoxidation of an olefin according to any of claims 1-12.

## Patentansprüche

1. Verfahren zur Epoxidation eines Olefins, welches Verfahren die folgenden Schritte umfaßt:
(a) Vorimprägnieren eines hochselektiven Epoxidationskatalysators auf Silberbasis mit einem organischen Halogenid,
(b) Führen eines Einsatzmaterials über den vorimprägnierten Katalysator, das frei von organischem Halogenid ist oder das ein organisches Halogenid in einer Konzentration von höchstens 2x10⁻⁴ Mol-% umfaßt, berechnet auf der Basis des Halogengehaltes in Bezug auf das Gesamteinsatzmaterial, während einer Dauer von über 16 Stunden bis zu 200 Stunden, und
(c) anschließendes Inkontaktbringen des Katalysators mit einem das Olefin, Sauerstoff und ein organisches Halogenid umfassenden.Einsatzmaterial, worin die Konzentration des organischen Halogenids wenigstens 0,2x10⁻⁴ Mol-% größer ist als die im Schritt (b) angewandte Konzentration, berechnet auf der Basis des Halogengehaltes in Bezug auf das Gesamteinsatzmaterial.

2. Verfahren nach Anspruch 1, worin der Katalysator, zusätzlich zu Silber, ein Gruppe IA-Metall und ein oder mehrere, die Selektivität fördernde Dotierungsmittel umfaßt, ausgewählt aus Rhenium, Molybdän, Wolfram und einer sulfat-, nitrat- oder nitritbildenden Verbindung.

3. Verfahren nach Anspruch 2, worin der Katalysator, zusätzlich zu Silber, Rhenium oder einer Verbindung davon, ein weiteres Metall oder eine Verbindung davon, ausgewählt aus der Gruppe von Gruppe IA-Metallen, Gruppe IIA-Metallen, Molybdän, Wolfram, Chrom, Titan, Hafnium, Zirkonium, Vanadium, Thallium, Thorium, Tantal, Niob, Gallium und Germanium und deren Gemischen, und gegebenenfalls einen Rhenium-Co-Promotor, der unter einem oder mehreren von Schwefel, Phosphor, Bor und deren Verbindungen ausgewählt sein kann, auf einem Trägermaterial umfaßt.

4. Verfahren nach einem der Ansprüche 1 bis 3, worin das organische Halogenid einen oder mehreren Chlorkohlenwasserstoffe oder Bromkohlenwasserstoffe umfaßt.

5. Verfahren nach einem der Ansprüche 1 bis 4, worin der Katalysator durch Inkontaktbringen des Katalysators mit einer Menge an dem organischen Halogenid im Bereich von 0,01 bis 50 mMol/kg Katalysator, insbesondere von 0,05 bis 20 mMol/kg Katalysator, im spezielleren von 0,1 bis 10 mMol/kg Katalysator, berechnet auf der Basis des Halogengehaltes des organischen Halogenids, bei einer Temperatur im Bereich von 180 bis 265°C, stärker bevorzugt im Bereich von 200 bis einschließlich 260°C, vorimprägniert wird.

6. Verfahren nach einem der Ansprüche 1 bis 5, worin das über den vorimprägnierten Katalysator geführte Einsatzmaterial das organische Halogenid in einer Konzentration im Bereich von 0,01x10⁴ bis höchstens 1,5x10⁻⁴ Mol-%, berechnet auf der Basis des Halogengehaltes in Bezug auf das Gesamteinsatzmaterial, umfaßt.

7. Verfahren nach einem der Ansprüche 1 bis 6, worin die Dauer im Bereich von 20 bis 60 Stunden liegt.

8. Verfahren nach einem der Ansprüche 1 bis 7, worin die Temperatur, bei der das Einsatzmaterial über den vorimprägnierten Katalysator geführt wird, höchstens 270°C, insbesondere im Bereich von 200 bis 265°C beträgt.

9. Verfahren nach einem der Ansprüche 1 bis 8, worin das über den vorimprägnierten Katalysator geführte Einsatzmaterial ein Olefin, insbesondere Ethylen, gegebenenfalls einen gesättigten Kohlenwasserstoff und Sauerstoff umfaßt und worin die Olefinmenge im Bereich von 5 bis 70 Mol-%, insbesondere von 10 bis 50 Mol-% liegt; die Menge des gesättigten Kohlenwasserstoffes im Bereich von 0 bis 70 Mol-%, insbesondere von 10 bis 60 Mol-% liegt; und die Sauerstoffmenge im Bereich von 0,5 bis 15 Mol-%, insbesondere von 1 bis 12 Mol-% liegt.

10. Verfahren nach einem der Ansprüche 1 bis 9, worin im Schritt (c) die Konzentration des organischen Halogenids wenigstens um 1x10⁻⁴ Mol-% höher ist als die im Schritt (b) angewandte Konzentration, berechnet auf der Basis des Halogengehaltes in Bezug auf das Gesamteinsatzmaterial.

11. Verfahren nach einem der Ansprüche 1 bis 10, worin im Schritt (c) das Einsatzmaterial das organische Halogenid bei einer Konzentration umfaßt, bei der die Selektivität auf das Olefinoxid bei ihrem Optimum liegt.

12. Verfahren nach einem der Ansprüche 1 bis 11, worin das Olefin Ethylen ist.

13. Verfahren zur Herstellung eines 1,2-Diols, 1,2-Diolethers oder eines Alkanolamins, umfassend ein Umwandeln eines O-lefinoxids in das 1,2-Diol, den 1,2-Diolether oder das Alkanolamin, worin das Olefinoxid nach einem Verfahren zur Epoxidation eines Olefins gemäß einem der Ansprüche 1 bis 12 erhalten worden ist.

## Revendications

1. Procédé pour l'époxydation d'une oléfine, lequel procédé comprend les étapes suivantes :
(a) on pré-imprègne un catalyseur d'époxydation hautement sélectif à base d'argent avec un halogénure organique,
(b) on fait passer sur le catalyseur pré-imprégné une charge qui est exempte d'halogénure organique ou qui comprend un halogénure organique en concentration d'au maximum 2 x 10⁻⁴% en mole, calculée sur la base de la teneur en halogène par rapport à la charge totale, sur une période de plus de 16 heures jusqu'à 200 heures, et
(c) on met ensuite en contact le catalyseur avec une charge comprenant l'oléfine, de l'oxygène et un halogénure organique, dans laquelle la concentration en halogénure organique est au moins 0,2 x 10⁻⁴% en mole supérieure à la concentration appliquée à l'étape (b), calculée sur la base de la teneur en halogène par rapport à la charge totale.

2. Procédé selon la revendication 1, dans lequel le catalyseur comprend, en plus de l'argent, un métal du groupe IA, et un ou plusieurs dopants renforçant la sélectivité choisis parmi le rhénium, le molybdène, le tungstène, et un composé formant du sulfate, du nitrate ou du nitrite.

3. Procédé selon la revendication 2, dans lequel le catalyseur comprend, en plus de l'argent, du rhénium ou un de ses composés, un autre métal ou un de ses composés, choisi dans le groupe des métaux du groupe IA, des métaux du groupe IIA, du molybdène, du tungstène, du chrome, du titane, de l'hafnium, du zirconium, du vanadium, du thallium, du thorium, du tantale, du niobium, du gallium et du germanium et de leurs mélanges, et éventuellement un co-promoteur de rhénium qui peut être choisi parmi un ou plusieurs parmi le soufre, le phosphore, le bore et leurs composés, sur un matériau de support.

4. Procédé selon l'une quelconque des revendications 1 à 3, dans lequel l'halogénure organique comprend un ou plusieurs chlorohydrocarbures ou bromohydrocarbures.

5. Procédé selon l'une quelconque des revendications 1 à 4, dans lequel le catalyseur est pré-imprégné en mettant en contact le catalyseur avec une quantité de l'halogénure organique dans la plage de 0,01 à 50 mmoles/kg de catalyseur, particulièrement 0,05 à 20 mmoles/kg de catalyseur, plus particulièrement 0,1 à 10 mmoles/kg de catalyseur, calculée sur la base de la teneur en halogène de l'halogénure organique, à une température dans la plage de 180 à 265°C, mieux encore dans la plage de 200 à 260°C, inclus.

6. Procédé selon l'une quelconque des revendications 1 à 5, dans lequel la charge passant sur le catalyseur pré-imprégné comprend l'halogénure organique en concentration dans la plage de 0,01 x 10⁻⁴ au maximum à 1, 5 x 10⁻⁴% en mole, calculée sur la base de la teneur en halogène par rapport à la charge totale.

7. Procédé selon l'une quelconque des revendications 1 à 6, dans lequel la période se situe dans la plage de 20 à 60 heures.

8. Procédé selon l'une quelconque des revendications 1 à 7, dans lequel la température est au maximum de 270°C, en particulier dans la plage de 200 à 265°C, lorsqu'on fait passer la charge sur le catalyseur pré-imprégné.

9. Procédé selon l'une quelconque des revendications 1 à 8, dans lequel la charge passant sur le catalyseur pré-imprégné comprend une oléfine, en particulier l'éthylène, éventuellement un hydrocarbure saturé, et de l'oxygène et dans lequel la quantité de l'oléfine se situe dans la plage de 5 à 70% en mole, en particulier de 10 à 50% en mole; la quantité de l'hydrocarbure saturé se situe dans la plage de 0 à 70% en mole, en particulier de 10 à 50% en mole; et la quantité d'oxygène se situe dans la plage de 0,5 à 15% en mole, en particulier de 1 à 12% en mole.

10. Procédé selon l'une quelconque des revendications 1 à 9, dans lequel, à l'étape (c), la concentration en halogénure organique est au moins 1 x 10⁻⁴% en mole supérieure à la concentration appliquée à l'étape (b), calculée sur la base de la teneur en halogène par rapport à la charge totale.

11. Procédé selon l'une quelconque des revendications 1 à 10, dans lequel, à l'étape (c), la charge comprend l'halogénure organique en concentration à laquelle la sélectivité envers l'oxyde d'oléfine est optimale.

12. Procédé selon l'une quelconque des revendications 1 à 11, dans lequel l'oléfine est l' éthylène.

13. Procédé pour produire un 1,2-diol, un éther de 1,2-diol ou une alcanolamine, comprenant la conversion d'un oxyde d'oléfine en 1,2-diol, en éther de 1,2-diol ou en alcanolamine, dans lequel l'oxyde d'oléfine a été obtenu par un procédé pour l'époxydation d'une oléfine selon l'une quelconque des revendications 1 à 12.
